(19) Europäisches Patentamt
European Patent Office
Office européen des brevets

(11) Veröffentlichungsnummer: **0 007 392**
**B1**

(12) # EUROPÄISCHE PATENTSCHRIFT

(45) Veröffentlichungstag der Patentschrift: **08.06.83**

(51) Int. Cl.³: **C 07 D 413/14,** C 07 D 417/14, D 06 L 3/12 // C07D213/78

(21) Anmeldenummer: **79101404.6**

(22) Anmeldetag: **08.05.79**

(54) **Stilbenverbindungen, Verfahren zu deren Herstellung und deren Verwendung als optische Aufheller.**

(30) Priorität: **10.05.78 DE 2820322**

(43) Veröffentlichungstag der Anmeldung: **06.02.80 Patentblatt 80/3**

(45) Bekanntmachung des Hinweises auf die Patenterteilung: **08.06.83 Patentblatt 83/23**

(84) Benannte Vertragsstaaten: **AT BE CH DE FR GB IT NL SE**

(56) Entgegenhaltungen: **CH-D-1 191 964** **FR-A-2 343 741**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Erckel, Rüdiger, Dr., Staufenstrasse 16, D-6239 Eppstein/Taunus (DE)**
Erfinder: **Rösch, Günter, Hohlweg 17, D-6232 Bad Soden am Taunus (DE)**

EP 0 007 392 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Stilbenverbindungen, Verfahren zu deren Herstellung und deren Verwendung als optische Aufheller

Aus der FR-A-2343741 und der CH-A-11919/64 sind bereits optische Aufheller aus der Reihe der 1-Benzoxazolyl-4'-oxdiazolyl-stilbene bekannt. Speziell die FR-A-2343741 beschreibt derartige 1-Benzoxazolyl-4'-oxdiazolyl-stilbene, die einen aryl-substituierten 1,2,4-Oxdiazolyl-3 oder 1,2,4-Oxdiazolyl-5-Ring tragen. Es wurde nun gefunden, dass man die Aufhellwirkung von Verbindungen dieses Typs noch steigern kann, wenn man den Arylsubstituenten des Oxdiazolylrings gegen heterocyclische Reste austauscht.

Gegenstand der Erfindung sind Verbindungen der allgemeinen Formel I

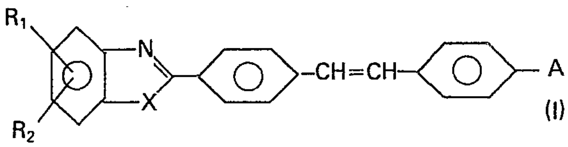

(I)

worin X, O oder S, $R_1$ und $R_2$ gleiche oder verschiedene Reste aus der Gruppe Wasserstoff-, Fluor- oder Chloratome, Phenyl-, niedere Alkyl-, niedere Alkoxy-, niedere Dialkylamino-, niedere Trialkylammonium-, Acylaminogruppen oder gegebenenfalls funktionell abgewandelte Carboxy- oder Sulfogruppen, wobei zwei benachbarte Reste $R_1$ und $R_2$ zusammen auch für eine Phenylen-, eine niedere Alkylen- oder eine 1,3-Dioxapropylengruppe stehen können, bedeuten und A eine Gruppe der Formel

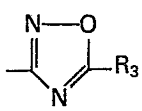 oder 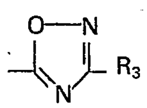

bedeutet, wobei $R_3$ eine α-, β- oder γ-Pyridyl-, Pyridylmethyl-, Thienyl-, Furanyl-, Benzofuranyl-, Pyrazinyl-, Pyrimidinyl- oder Pyridazinylgruppe darstellt.

Von besonderem Interesse sind die Verbindungen der Formel I, wobei A, $R_1$ und $R_2$ die oben genannten Bedeutungen haben, X ein Sauerstoffatom und $R_3$ eine α-, β- oder γ-Pyridyl-, Pyridylmethylen-, Thiophenyl-, Pyrazinyl-, Pyrimidinyl- oder Pyridazinylgruppe bedeutet.

Bevorzugt sind auch solche Verbindungen der allgemeinen Formel I, worin X Sauerstoff, $R_1$ und $R_2$ in 5-, 6- und 7-Stellung Wasserstoff- oder Chloratome, $(C_1-C_4)$ Alkyl, Phenyl oder zusammen einen ankondensierten Phenylring und $R_3$ in der Gruppe A eine α- oder β-Pyridyl-, Thiophenyl-, Furanyl-, Benzofuranyl- oder Pyridylmethylengruppe bedeutet.

Als weitere Untergruppe sind diejenigen Verbindungen der allgemeinen Formel I von besonderem Interesse, worin X ein Sauerstoffatom, $R_1$ in 5-Stellung ein Wasserstoff- oder Chloratom, eine Methyl- oder Phenylgruppe, $R_2$ ein Wasserstoffatom oder $R_1$ und $R_2$ beide eine Methylgruppe in 5,6- oder 5,7-Stellung und $R_3$ in der Gruppe A eine α- oder β-Pyridyl-, Thiophenyl-, Furanyl-, Benzofuranyl- oder Pyridylmethylengruppe bedeutet.

Unter dem Begriff «funktionell abgewandelte Carboxy- oder Sulfogruppen» sind folgende zu verstehen:

Cyano-, Carbonsäureester-, Carbonsäureamid-, Mono- und Dialkylcarbonamid-, Sulfonsäureester- und Mono- bzw. Dialkylsulfonamidgruppen.

Im einzelnen kommen beispielsweise für $R_1$ und $R_2$ folgende Reste in Frage: Methyl, Äthyl- n- oder i-Propyl- n- oder i-Butyl, Pentyl, Hexyl, Methoxy, Äthoxy, Propoxy, Butoxy, Pentoxy, Hexoxy, Dimethylamino, Diäthylamino, Trimethylammonium, Triäthylammonium, Acetylamino, Cyano, $-SO_3H$, Carboxyl, Carbo-methoxy, -äthoxy, -propoxy, -butoxy und die entsprechenden Gruppen in der Reihe der Sulfonsäurealkylestergruppen, Methyl-, Äthyl-, Propyl-, Butyl-carbonamid und die entsprechenden Gruppen in der Reihe der Alkylsulfonamide und die entsprechenden Dialkylcarbonamidgruppen bzw. -sulfonamidgruppen. Zwei benachbarte Gruppen $R_1$ und $R_2$ können zusammen auch einen ankondensierten Phenyl- oder Cyclohexylring bilden. Unter der Bedeutung von X sind all diejenigen Verbindungen, die die benzoxazolylgruppe enthalten (X=O).

Gegenstand der Erfindung ist ferner ein Verfahren zur Herstellung der Verbindungen der Formel I, dadurch gekennzeichnet, dass man eine Verbindung der allgemeinen Formel II

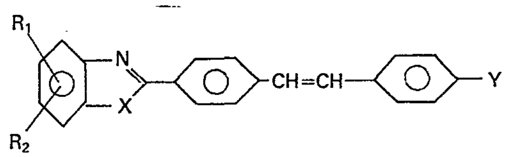

(II)

mit einer Verbindung der allgemeinen Formel III

$$R_3-Z \qquad (III)$$

umsetzt, wobei $R_1$, $R_2$, X und $R_3$ die zuvor angegebenen Bedeutungen haben und Y eine Gruppe der Formel IV

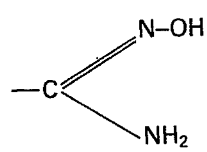

(IV)

bedeutet, wobei Z gleichzeitig eine Gruppe der Formel V

$$-COCl \qquad (V)$$

oder Y bedeutet eine Gruppe der Formel V und Z ist gleichzeitig eine Gruppe der Formel IV.

Im ersten Fall erhält man solche Verbindungen der Formel I, die eine 1,2,4-Dioxazolyl-3-Gruppe enthalten und im zweiten Fall enthalten die Verbindungen die 1,2,4-Dioxazolyl-5-Gruppe. Die Umsetzung erfolgt vorzugsweise in Gegenwart eines säurebindenden Mittels in einem inerten Lösungsmittel bei Temperaturen von 20–200°C. Als Lösungsmittel für die Reaktion eignen sich z. B. Chlorbenzol, Di- oder Trichlorbenzol und insbesondere Dimethylformamid, N-Methylpyrrolidon, Dimethylsulfoxid oder Nitrobenzol. Als säurebindende Mittel sind beispielsweise Natriumcarbonat, Kaliumcarbonat, Calciumcarbonat, Triäthylamin oder Äthyldiisopropylamin verwendbar.

Die Verbindungen der Formel II, wobei Y eine Gruppe der Formel IV bedeutet, erhält man aus den entsprechenden Nitrilen durch Umsetzung mit Hydroxylamin, vorzugsweise in Alkoholen oder N-Methylpyrrolidon. Die entsprechenden Nitrile sind literaturbekannt bzw. können nach literaturbekannten Verfahren dargestellt werden (JA Sho 42-21013, US 3577411, DT-OS 2000027). Beispielweise lässt sich die entsprechend substituierte Benzoxazolylstilbencarbonsäure nach an sich literaturbekannten Verfahren über das Säurechlorid in das Amid überführen und dieses ebenfalls nach an sich literaturbekannten Verfahren durch Umsetzung mit einem wasserabspaltenden Mittel zu dem Nitril umsetzen. Die Ausgangsverbindungen der Formel II, wo Y eine Gruppe der Formel IV bedeutet, lassen sich auch nach dem in Chem. Rev. 62 (1962), S. 155 ff angegebenen Verfahren herstellen. Nach diesem Verfahren lassen sich auch in analoger Weise die Ausgangsverbindungen der Formel III herstellen, in denen Z eine Gruppe der Formel IV darstellt. Die Ausgangsverbindungen der Formel II, wo Y eine Gruppe der Formel V ist, erhält man durch folgende, dem Fachmann an sich bekannte Reaktionsstufen:

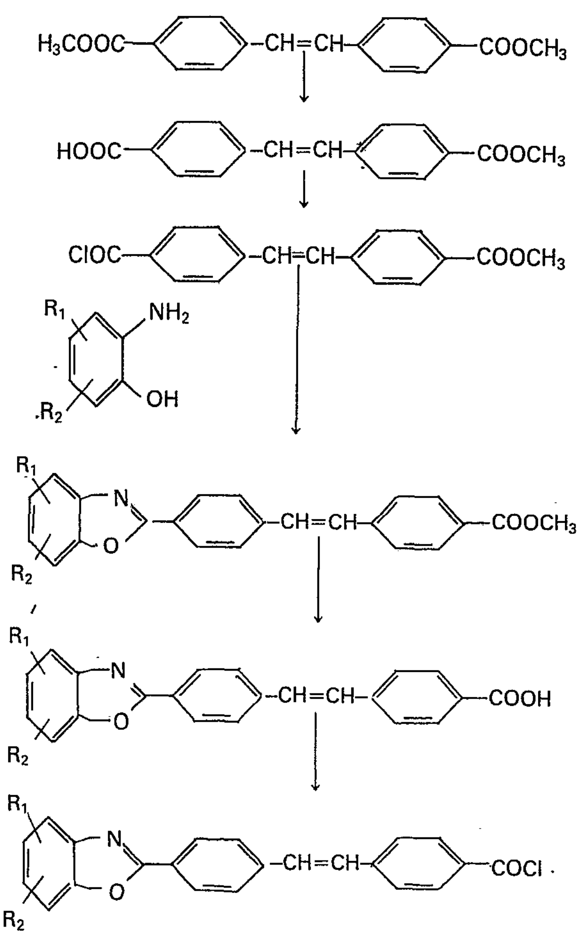

An den Reaktionsprodukten der vorstehenden Verfahren können noch weitere an sich bekannte Umwandlungen vorgenommen werden, z. B. solche Umwandlungen, die ausgehend von sulfo- oder carboxy-haltigen Molekülen zu Verbindungen mit funktionell abgewandelten Sulfo- oder Carboxygruppen führen bzw. die Umwandlungen solcher Gruppen in andere Gruppen dieser Art oder in die freien Säuren. Weiterhin können z. B. auch in bekannter Weise Chlormethylgruppen eingeführt oder Methylgruppen oxydiert werden. Ebenso gelingen Halogenierungen und weitere Umsetzungen der eingeführten Halogenatome, so z. B. der Austausch von Chlor oder Brom gegen die Amin-Funktion.

Die neuen Verbindungen der Formel I sind nahezu farblose, fluoreszierende Substanzen, die sich als optische Aufheller eignen.

Als aufzuhellende Substrate seien beispielsweise folgende Materialien genannt: Lacke, natürliche oder synthetische Fasern, wie z. B. solche aus natürlicher oder regenerierter Cellulose, Acetylcellulose, natürlichen und synthetischen Polyamiden wie Wolle, Polyamid-6 und -6,6-Polyestern, Polyolefinen, Polyvinylchlorid, Polyvinylidenchlorid, Polystyrol oder Polyacrylnitril sowie Folien, Filme, Bänder oder Formkörper aus solchen Materialien.

Die in Wasser unlöslichen erfindungsgemässen Verbindungen können gelöst in organischen Lösungsmitteln zum Einsatz kommen oder in wässriger Dispersion, vorteilhaft unter Zuhilfenahme eines Dispergierungsmittels, eingesetzt werden. Als Dispergierungsmittel kommen beispielsweise Seifen, Polyglykoläther, die sich von Fettalkoholen, Fettaminen oder Alkylphenolen ableiten, Cellulose-sulfitablaugen oder Kondensationsprodukte von gegebenenfalls alkylierten Naphthalinsulfonsäuren mit Formaldehyd in Frage.

Verbindungen der allgemeinen Formel I können auch Waschmitteln zugesetzt werden. Diese können die üblichen Füll- und Hilfsstoffe, wie Alkalisilikate, Alkali-phosphate und -polymetaphosphate, Alkaliborate, Alkalisalze der Carboxymethylcellulosen, Schaumstabilisatoren, wie Alkanolamide höherer Fettsäuren oder Komplexbildner, wie lösliche Salze der Äthylendiamintetraessigsäure oder Diäthylentriaminpentaessigsäure, sowie chemische Bleichmittel, wie Perborate oder Percarbonate, enthalten.

Die Aufhellung des Fasermaterials mit der wässrigen oder ev. organischen Aufhellerflotte erfolgt entweder im Ausziehverfahren bei Temperaturen von vorzugsweise etwa 20 bis etwa 150°C oder unter Thermosolierbedingungen, wobei das Textilmaterial mit der Aufhellerlösung bzw. -dispersion imprägniert oder besprüht und zwischen Walzen auf einen Restfeuchtigkeitsgehalt von etwa 50 bis etwa 120% abgequetscht wird. Anschliessend wird das Textilmaterial etwa 10 bis etwa 300 sek einer Temperaturbehandlung, vorzugsweise mittels Trockenhitze bei etwa 120 bis etwa 240°C unterzogen. Dieser Thermosolierprozess kann auch mit anderen Ausrüstungsoperationen, z. B. der Ausrüstung mit Kunstharzen zum

Zwecke der Pflegeleichtigkeit, kombiniert werden.

Ferner können die erfindungsgemässen Verbindungen hochmolekularen organischen Materialien vor bzw. während deren Verformung zugesetzt werden. So kann man sie beispielsweise bei der Herstellung von Filmen, Folien, Bändern oder Formkörpern den Pressmassen beifügen oder vor dem Verspinnen in der Spinnmasse lösen. Geeignete Verbindungen können auch vor der Polykondensation oder Polymerisation, wie im Falle von Polyamid-6, Polyamid-6,6 oder linearen Estern vom Typ des Polyäthylenglykolterephthalats, den niedermolekularen Ausgangsmaterialien zugesetzt werden.

Erfindungsgemässe Verbindungen, die durch eine oder vorzugsweise zwei Carboxy- oder Carbalkoxygruppen substituiert sind, können an lineare Polyestermoleküle und synthetische Polyamide durch eine Ester- oder Amidbindung gebunden werden, wenn sie unter geeigneten Bedingungen diesen Materialien oder bevorzugt deren Ausgangsstoffen zugesetzt werden. Auf diese Weise durch eine chemische Bindung im Substrat verankerte Aufheller zeichnen sich durch eine ausserordentlich hohe Sublimier- und Lösungsmittelechtheit aus.

Die Menge der erfindungsgemäss zu verwendenden Verbindungen der allgemeinen Formel I, bezogen auf das optisch aufzuhellende Material, kann je nach Einsatzgebiet und gewünschtem Effekt in weiten Grenzen schwanken. Sie kann durch Versuche leicht ermittelt werden und liegt im allgemeinen zwischen etwa 0,01 und etwa 2%.

Angaben über Teile und Prozente beziehen sich im folgenden, sofern nicht anders vermerkt, auf das Gewicht.

Beispiel 1

In 100 ml Dimethylformamid werden 3,4 g (0,024 Mol) Pyridin-4-amidoxim vorgelegt und 7,2 g (0,02 Mol) 4'-Benzoxazolyl-2-stilben-4-carbonsäurechlorid eingetragen. Anschliessend wird 1 h bei Raumtemperatur und 2 h am Rückfluss gerührt. Es wird bei Raumtemperatur abgesaugt und der Filterrückstand mit Dimethylformamid und Methanol nachgewaschen. Nach dem Trocknen erhält man 7,8 g (87,6% d. Th.) der Verbindung der Formel

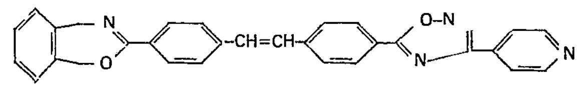

in Form eines hellgelben Pulvers, welche nach Umkristallisieren aus N-Methylpyrrolidin unter Klären mit Tierkohle einen flüssig-kristallinen Übergang bei 274–275°C zeigt und bei 281°C schmilzt.

ber. C 76,0%, H 4,1%, N 12,7%
gef. C 75,8%, H 4,2%, N 12,8%
Absorption: max = 367 nm
(in DMF) = 74500
Fluoreszenz: max = 432 nm
(in DMF)

Das eingesetzte Pyridin-4-amidoxim erhält man folgendermassen:

70 g (1 Mol) Hydroxylammoniumchlorid werden in 500 ml Methanol vorgelegt und eine Lösung von 54 g (1 Mol) Natriummethylat in 500 ml Methanol zugegeben. Nach etwa 30 min wird vom Natriumchlorid abfiltriert, zum Filtrat 41,6 g (0,4 Mol) Pyridin-4-carbonsäurenitril zugegeben und 24 h am Rückfluss gerührt. Anschliessend wird das Methanol abdestilliert und der Rückstand unter Klären mit Bleicherde aus 2000 ml Isopropanol umkristallisiert. Man erhält 49,1 g (89,5% d. Th. bzw. auf eingesetztes Nitril) Pyridin-4-amidoxim mit einem Schmelzpunkt von 207–208°C, welches ohne weitere Reinigung umgesetzt wird.

In analoger Weise werden die in der folgenden Tabelle aufgeführten Verbindungen erhalten.

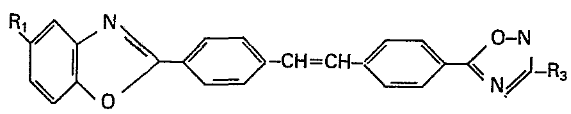

| 1 fd: Nr. | $R_1$ | $R_3$ | Fp* (°C) | Absorption (gem. in DMF) max (nm) | ε | Fluoreszenz gemessen in DMF) max (nm) |
|---|---|---|---|---|---|---|
| 2 | $CH_3$ | 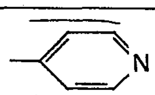 | 261–263 | 368 | 75200 | 442 |
| 3 | H | 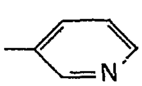 | 260–262 | 367 | – | 431 |
| 4 | $CH_3$ | 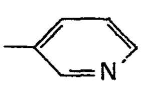 | 270–273 | 369 | 76700 | 483 |
| 5 | H | 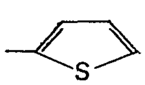 | 261–262 | 367 | 74200 | 431 |

| 1 fd: Nr. | $R_1$ | $R_3$ | Fp* (°C) | Absorption (gem. in DMF) max (nm) | ε | Fluoreszenz gemessen in DMF) max (nm) |
|---|---|---|---|---|---|---|
| 6 | $CH_3$ | 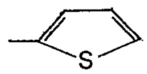 | 251–260 | 368 | 76100 | 436 |
| 7 | H | 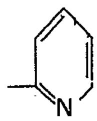 | 297–298 | 368 | 74700 | 429 |
| 8 | $CH_3$ | 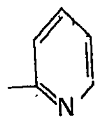 | 299–300 | 369 | 76300 | 435 |
| 9 | H | 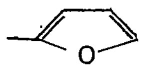 | 267–268 | 367 | 74700 | 431 |
| 10 | H | 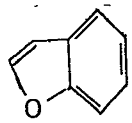 | 273–274 | 367 | – | 433 |
| 11 | H | $-CH_2-$ 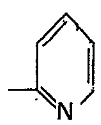 | 260–267 | 366 | 72300 | 428 |

*) angegeben ist der Bereich des flüssig-kristallinen Übergangs

**Patentansprüche für die Vertragsstaaten: BE, CH, DE, FR, GB, IT, NL, SE**

1. Verbindungen der allgemeinen Formel I

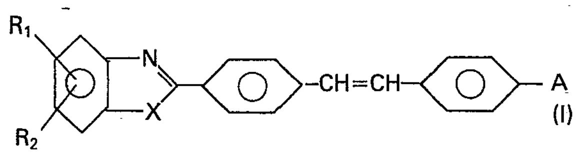

(I)

worin X, O oder S, $R_1$ und $R_3$ gleiche oder verschiedene Reste aus der Gruppe Wasserstoff-, Fluor- oder Chloratome, Phenyl-, niedere Alkyl-, niedere Alkoxy-, niedere Dialkylamino-, niedere Trialkylammonium-, Acylaminogruppen oder gegebenenfalls funktionell abgewandelte Carboxy- oder Sulfogruppen, wobei zwei benachbarte Reste $R_1$ und $R_2$ zusammen auch für eine Phenylen-, eine niedere Alkylen- oder eine 1,3-Dioxapropylengruppe stehen können, bedeuten und A eine Gruppe der Formel

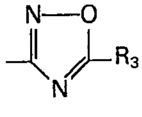 oder 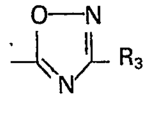

bedeutet, wobei $R_3$ eine α-, β- oder γ-Pyridyl-, Pyridylmethyl-, Thienyl-, Furanyl-, Benzofuranyl-, Pyrazinyl-, Pyrimidinyl- oder Pyridazinylgruppe darstellt.

2. Verbindungen gemäss Anspruch 1, wobei A, $R_1$ und $R_2$ die in Anspruch 1 angegebenen Bedeutungen haben, X ein Sauerstoffatom und $R_3$ eine α-, β oder γ-Pyridyl-, Pyridylmethyl-, Thienyl-, Pyrazinyl-, Pyrimidinyl- oder Pyridazinylgruppe bedeutet.

3. Verbindungen gemäss Anspruch 1, wobei X Sauerstoff, $R_1$ und $R_2$ in 5- und 7-Stellung Wasserstoff- oder Chloratome $(C_1-C_4)$Alkyl, Phenyl oder zusammen einen ankondensierten Phenylring und $R_3$ eine α- oder β-Pyridyl-, Thienyl-, Furanyl-, Benzofuranyl- oder Pyridylmethylgruppe bedeutet.

4. Verbindungen gemäss Anspruch 1, wobei X ein Sauerstoffatom, $R_1$ in 5-Stellung ein Wasserstoff- oder Chloratom, eine Methyl- oder Phenylgruppe, $R_2$ ein Wasserstoffatom oder $R_1$ und $R_2$ beide eine Methylgruppe in 5,6- oder 5,7-Stellung und $R_3$ in der Gruppe A eine α- oder β-Pyridyl-, Thienyl-, Furanyl-, Benzofuranyl- oder Pyridylmethylgruppe bedeutet.

5. Verfahren zur Herstellung von Verbindungen gemäss Anspruch 1 bis 4, dadurch gekennzeichnet, dass man eine Verbindung der allgemeinen Formel II

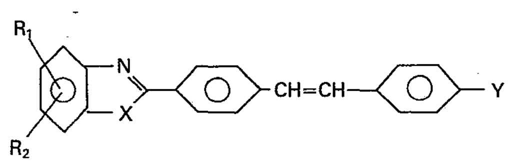

(II)

mit einer Verbindung der allgemeinen Formel III

$R_3-Z$ (III)

umsetzt, wobei $R_1$, $R_2$, X und $R_3$ die in Anspruch 1 bis 4 angegebenen Bedeutungen haben und Y eine Gruppe der Formel IV

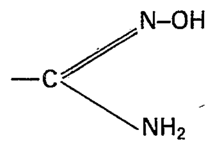

(IV)

bedeutet, wobei Z gleichzeitig eine Gruppe der Formel V

$-COCl$ (V)

darstellt oder Y bedeutet eine Gruppe der Formel V und Z ist gleichzeitig eine Gruppe der Formel IV.

6. Verfahren nach Anspruch 5, dadurch gekennzeichnet, dass man die Umsetzung in Gegenwart eines säurebindenden Mittels in einem inerten Lösungsmittel bei Temperaturen von 20 bis 200°C durchführt.

7. Verwendung der Verbindungen gemäss Anspruch 1 bis 4 als optische Aufheller.

**Patentansprüche für den Vertragsstaat: AT**

1. Verfahren zur Herstellung von Verbindungen der allgemeinen Formel I

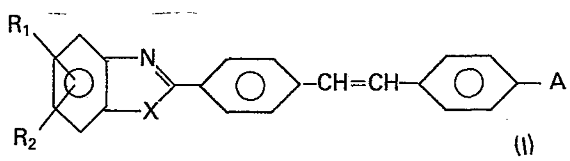

(I)

worin X, O oder S, $R_1$ und $R_2$ gleiche oder verschiedene Reste aus der Gruppe Wasserstoff-, Fluor- oder Chloratome, Phenyl-, niedere Alkyl-, niedere Alkoxy-, niedere Dialkylamino-, niedere Trialkylammonium-, Acylaminogruppen oder gegebenenfalls funktionell abgewandelte Carboxy- oder Sulfogruppen, wobei zwei benachbarte Reste $R_1$ und $R_2$ zusammen auch für eine Phenylen-, eine niedere Alkylen- oder eine 1,3-Dioxapropylengruppe stehen können, bedeuten und A eine Gruppe der Formel

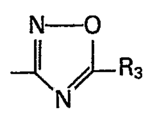 oder 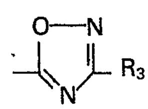

bedeutet, wobei $R_3$ eine α-, β oder γ-Pyridyl-, Pyridylmethyl-, Thienyl-, Furanyl-, Benzofuranyl-, Pyrazinyl-, Pyrimidinyl- oder Pyridazinylgruppe darstellt, dadurch gekennzeichnet, dass man eine Verbindung der allgemeinen Formel II

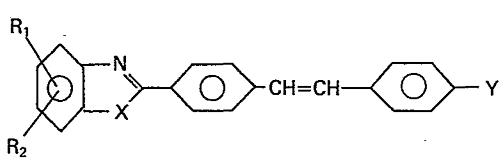

(II)

mit einer Verbindung der allgemeinen Formel III

$R_3{-}Z$ (III)

umsetzt, wobei $R_1$, $R_2$, X und $R_3$ die in Anspruch 1 bis 4 angegebenen Bedeutungen haben und Y eine Gruppe der Formel IV

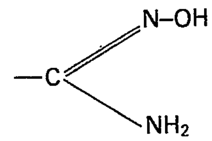

(IV)

bedeutet, wobei Z gleichzeitig eine Gruppe der Formel V

$-COCl$ (V)

darstellt oder Y bedeutet eine Gruppe der Formel V und Z ist gleichzeitig eine Gruppe der Formel IV.

2. Verfahren nach Anspruch 1, dadurch gekennzeichnet, dass Verbindungen der Formel I hergestellt werden, wobei A, $R_1$ und $R_2$ die in Anspruch 1 angegebenen Bedeutungen haben, X ein Sauerstoffatom und $R_3$ eine α-, β- oder γ-Pyridyl-, Pyridylmethyl-, Thienyl-, Pyrazinyl-, Pyrimidinyl- oder Pyridazinylgruppe bedeutet.

3. Verfahren nach Anspruch 1, dadurch gekennzeichnet, dass Verbindungen der Formel I hergestellt werden, wobei X Sauerstoff, $R_1$ und $R_2$ in 5- und 7-Stellung Wasserstoff- oder Chloratome $(C_1-C_4)$Alkyl, Phenyl oder zusammen einen ankondensierten Phenylring und $R_3$ eine α- oder β-Pyridyl-, Thienyl-, Furanyl-, Benzofuranyl- oder Pyridylmethylgruppe bedeutet.

4. Verfahren nach Anspruch 1, dadurch gekennzeichnet, dass Verbindungen der Formel I hergestellt werden, wobei X ein Sauerstoffatom, $R_1$ in 5-Stellung ein Wasserstoff- oder Chloratom, eine Methyl- oder Phenylgruppe, $R_2$ ein Wasserstoffatom oder $R_1$ und $R_2$ beide eine Methylgruppe in 5,6- oder 5,7-Stellung und $R_3$ in der Gruppe A eine α- oder β-Pyridyl-, Thienyl-, Furanyl-, Benzofuranyl- oder Pyridylmethylgruppe bedeutet.

5. Verfahren nach Anspruch 1, dadurch gekennzeichnet, dass man die Umsetzung in Gegenwart eines säurebindenden Mittels in einem inerten Lösungsmittel bei Temperaturen von 20 bis 200°C durchführt.

6. Verwendung der Verbindungen der Formel I, die gemäss Anspruch 1 hergestellt werden, als optische Aufheller.

**Claims for the contracting state: AT**

1. A process for the manufacture of compounds of the general formula I

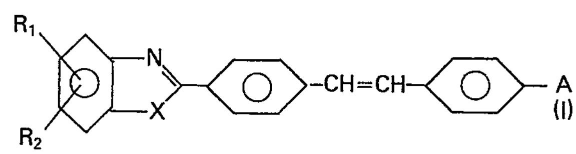

(I)

in which X is O or S; $R_1$ and $R_2$, being identical or different, each are radicals selected from the group of hydrogen, fluorine or chlorine atoms, phenyl, lower alkyl, lower alkoxy, lower dialkylamino, lower trialkylammonium, acylamino

groups, or optionally functionally modified carboxy or sulfo groups, two adjacent radicals $R_1$ and $R_2$ optionally forming together a phenylene, a lower alkylene or a 1,3-dioxapropylene group; and A is a group of the formulae

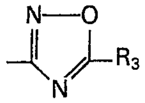 oder 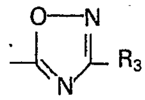

in which $R_3$ is an alpha-, beta- or gamma-pyridyl, pyridylmethyl, thienyl, furanyl, benzofuranyl, pyrazinyl, pyrimidinyl or pyridazinyl group, which is characterized by reacting a compound of the formula II

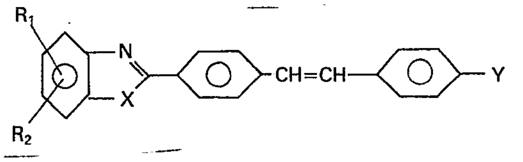 (II)

with a compound of the formula III

$$R_3\text{–}Z \qquad \text{(III)}$$

in which formulae $R_1$, $R_2$, X and $R_3$ are as defined in claims 1 to 4 and Y is a group of the formula IV

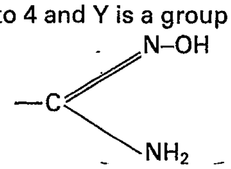 (IV)

Z simultaneously being a group of the formula V

$$\text{–COCl} \qquad \text{(V)}$$

or Y is a group of the formula V and Z is simultaneously a group of the formula IV.

2. A process as claimed in claim 1, which is characterized by preparing compounds of the formula I, wherein A, $R_1$ and $R_2$ are as defined in claim 1, X is oxygen and $R_3$ is an alpha-, beta- or gamma-pyridyl, pyridylmethyl, thienyl, pyrazinyl, pyrimidinyl or pyridazinyl group.

3. A process as claimed in claim 1, which is characterized by preparing compounds of the formula I, wherein X is oxygen; $R_1$ and $R_2$ in 5- or 7-position each are a hydrogen or chlorine atom, $(C_1-C_4)$-alkyl, phenyl, or together a fused phenyl ring; and $R_3$ is an alpha- or beta-pyridyl, thienyl, furanyl, benzofuranyl oder pyridylmethyl group.

4. A process as claimed in claim 1, which is characterized by preparing compounds of the formula I, wherein X is an oxygen atom; $R_1$ in 5-position is a hydrogen or chlorine atom, a methyl or phenyl group; $R_2$ is a hydrogen atom; or $R_1$ and $R_2$ each are a methyl group in 5,6- or 5,7-position; and $R_3$ in the A group is an alpha- or beta-pyridyl, thienyl, furanyl, benzofuranyl, or pyridylmethyl group.

5. The process as claimed in claim 1, wherein the reaction is carried out in the presence of an acid-binding agent in an inert solvent at a temperature of from 20 to 200°C.

6. Use of the compounds of formula I, prepared according to claim 1 as optical brighteners.

**Revendications pour l'Etat contractant: AT**

1. Procédé de préparation de composés répondant à la formule générale I:

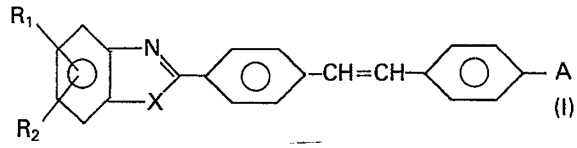 (I)

dans laquelle X représente O ou S, $R_1$ et $R_2$ représentent chacun, indépendamment l'un de l'autre, un atome d'hydrogène, de fluor ou de chlore, un radical phényle, un radical alkyle inférieur, un radical alcoxy inférieur, un radical dialkylamino inférieur, un radical trialkyl-ammonium inférieur, un radical acylamino ou un groupe carboxy ou sulfo éventuellement sous la forme d'un dérivé fonctionnel, deux radicaux $R_1$ et $R_2$ voisins pouvant également former ensemble un radical phénylène, alkylène inférieur ou dioxa-1,3 propylène, et A représente un radical répondant à l'une ou à l'autre des formules:

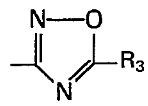 oder 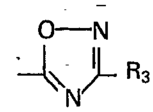

dans lesquelles $R_3$ représente un radical α-pyridyle, β-pyridyle, γ-pyridyle, pyridylméthyle, thiényle, furyle, benzofuryle, pyrazinyle, pyrimidinyle ou pyridazinyle, procédé caractérisé en ce qu'on fait réagir un composé de formule générale II:

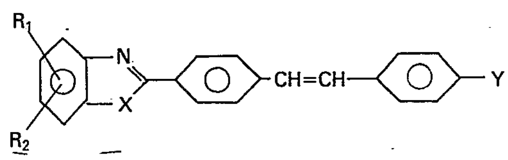 (II)

avec un composé de formule générale III:

$$R_3\text{–}Z \qquad \text{(III)}$$

formules dans lesquelles $R_1$, $R_2$, X et $R_3$ ont les significations indiquées ci-dessus et Y représente un radical de formule IV:

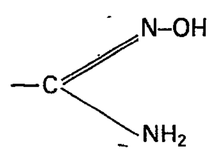 (IV)

auquel cas Z représente en même temps un radical de formule V

$$\text{–COCl} \qquad \text{(V)}$$

ou Y représente un radical de formule V et Z représente en même temps un radical de formule IV.

2. Procédé selon la revendication 1 caractérisé en ce qu'on prépare des composés de formule I dans lesquels A, $R_1$ et $R_2$ ont les significations données à la revendication 1, X représente un atome d'oxygène et $R_3$ représente un radical α-

pyridyle, β-pyridyle, γ-pyridyle, pyridylméthyle, thiényle, pyrazinyle, pyrimidinyle ou pyridazinyle.

3. Procédé selon la revendication 1, caractérisé en ce qu'on prépare des composés de formule I dans lesquels X représente l'oxygène, $R_1$ et $R_2$ se trouvent en position 5 et en position 7 et représentent chacun un atome d'hydrogène ou de chlore, un radical alkyle en $C_1-C_4$ ou un radical phényle ou forment ensemble un noyau benzénique condensé, et $R_3$ représente un radical α-pyridyle, β-pyridyle, thiényle, furyle, benzofuryle ou pyridylméthyle.

4. Procédé selon la revendication 1, caractérisé en ce qu'on prépare des composés de formule I dans lesquels X représents un atome d'oxygène, $R_1$ occupe la position 5 et représente un atome d'hydrogène ou de chlore, un radical méthyle ou un radical phényle, $R_2$ représente un atome d'hydrogène, ou $R_1$ et $R_2$ représente l'un et l'autre un radical méthyle en position 5,6 ou 5,7, et $R_3$ dans le radical A représente un radical α-pyridyle, β-pyridyle, thiényle, furyle, benzofuryle ou pyridylméthyle.

5. Procédé selon la revendication 1, caractérisé en ce qu'on effectue la réaction en présence d'un accepteur d'acides, dans un solvant inerte, à des températures de 20 à 200°C.

6. Application des composés de formule I qui ont été préparés selon la revendication 1, comme azureurs optiques.

**Revendications pour les Etats contractants: BE, CH, DE, FR, GB, IT, NL, SE**

1. Composés répondant à la formule générale I

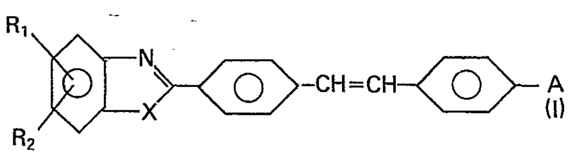

(I)

dans laquelle X représente O ou S, $R_1$ et $R_2$ représentent chacun, indépendamment l'un de l'autre, un atome d'hydrogène, de fluor ou de chlore, un radical phényle, un radical alkyle inférieur, un radical alcoxy inférieur, un radical dialkylamino inférieur, un radical trialkyl-ammonium inférieur, un radical acylamino ou un groupe carboxy ou sulfo éventuellement sous la forme d'un dérivé fonctionnel, deux radicaux $R_1$ et $R_2$ voisins pouvant également former ensemble un radical phénylène, alkylène inférieur ou dioxa-1,3 propylène, et A représente un radical répondant à l'une ou à l'autre des formules:

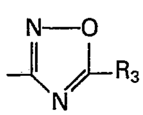 oder 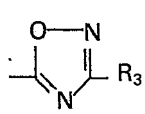

dans lesquelles $R_3$ représente un radical α-pyridyle, β-pyridyle, γ-pyridyle, pyridylméthyle, thiényle, furyle, benzofuryle, pyrazinyle, pyrimidinyle ou pyridazinyle.

2. Composés selon la revendication 1, dans lesquels A, $R_1$ et $R_2$ ont les significations données à la revendication 1, X représente un atome d'oxygène et $R_3$ représente un radical α-pyridyle, β-pyridyle, γ-pyridyle, pyridylméthyle, thiényle, pyrazinyle, pyrimidinyle ou pyridazinyle.

3. Composés selon la revendication 1, dans lesquels X représente l'oxygène, $R_1$ et $R_2$ sont aux positions 5 et 7 et représentent chacun un atome d'hydrogène ou de chlore, un radical alkyle en $C_1-C_4$ ou un radical phényle ou forment ensemble un noyau benzénique condensé, et $R_3$ représente un radical α-pyridyle, β-pyridyle, thiényle, furyle, benzofuryle ou pyridylméthyle.

4. Composés selon la revendication 1 dans lesquels X représente un atome d'oxygène, $R_1$ est en position 5 et représente un atome d'hydrogène ou de chlore, un radical méthyle ou un radical phényle, $R_2$ représente un atome d'hydrogène, ou $R_1$ et $R_2$ représente l'un et l'autre un radical méthyle en position 5,6 ou 5,7, et $R_3$ dans le radical A représente un radical α-pyridyle, β-pyridyle, thiényle, furyle, benzofuryle ou pyridylméthyle.

5. Procédé de préparation de composés selon l'une quelconque des revendications 1 à 4, caractérisé en ce qu'on fait réagir un composé de formule générale II

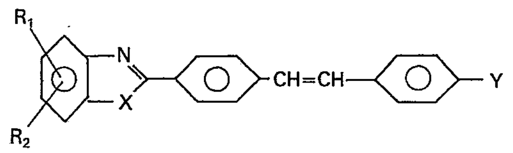 (II)

avec un composé de formule générale III

$R_3$-Z (III)

formules dans lesquelles $R_1$, $R_2$, X et $R_3$ ont les significations données dans les revendications 1 à 4 et Y représente un radical de formule IV

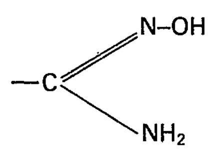 (IV)

auquel cas Z représente en même temps un radical de formule V

-COCl (V)

ou Y représente un radical de formule V et Z représente en même temps un radical de formule IV.

6. Procédé selon la revendication 5 caractérisé en ce qu'on effectue la réaction en présence d'un accepteur d'acides, dans un solvant inerte, à des températures de 20 à 200°C.

7. Application des composés selon l'une quelconque des revendications 1 à 4 comme azureurs optiques.

**Claims for the contracting states: BE, CH, DE, FR, GB, IT, NL, SE**

1. Compounds of the general formula I

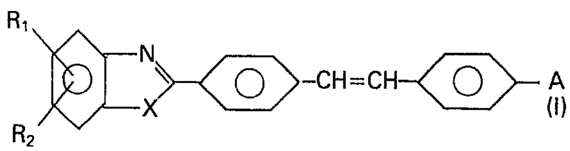

(I)

in which X is O or S; $R_1$ and $R_2$, being identical or different, each are radicals selected from the group of hydrogen, fluorine or chlorine atoms, phenyl, lower alkyl, lower alkoxy, lower dialkylamino, lower trialkylammonium, acylamino groups, or optionally functionally modified carboxy or sulfo groups, two adjacent radicals $R_1$ and $R_2$ optionally forming together a phenylene, a lower alkylene or a 1,3-dioxapropylene group; and A is a group of the formulae

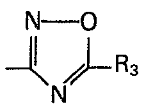 oder 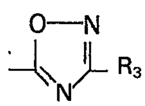

in which $R_3$ is an alpha-, beta- or gamma-pyridyl, pyridylmethyl, thienyl, furanyl, benzofuranyl, pyrazinyl, pyrimidinyl or pyridazinyl group.

2. Compounds as claimed in claim 1, wherein A, $R_1$ and $R_2$ are as defined in claim 1, X is oxygen and $R_3$ is an alpha-, beta- or gamma-pyridyl, pyridylmethyl, thienyl, pyrazinyl, pyrimidinyl or pyridazinyl group.

3. Compounds as claimed in claim 1, wherein X is oxygen; $R_1$ and $R_2$ in 5- or 7-position each are a hydrogen or chlorine atom, $(C_1-C_4)$-alkyl, phenyl, or together a fused phenyl ring; and $R_3$ is an alpha- or beta-pyridyl, thienyl, furanyl, benzofuranyl or pyridylmethyl group.

4. Compounds as claimed in claim 1, wherein X is an oxygen atom; $R_1$ in 5-position is a hydrogen or chlorine atom, a methyl or phenyl group; $R_2$ is a hydrogen atom; or $R_1$ and $R_2$ each are a methyl group in 5,6- or 5,7-position; and $R_3$ in the A group is an alpha- or beta-pyridyl, thienyl, furanyl, benzofuranyl or pyridylmethyl group.

5. A process for the manufacture of compounds as claimed in claims 1 to 4, which comprises reacting a compound of the formula II

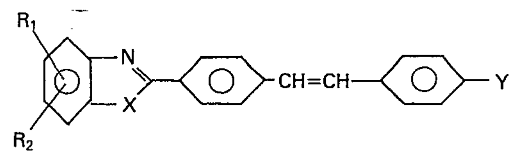

(II)

with a compound of the formula III

$$R_3-Z \qquad (III)$$

in which formulae $R_1$, $R_2$, X and $R_3$ are as defined in claims 1 to 4 and Y is a group of the formula IV

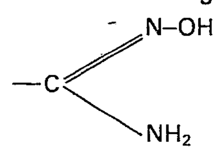

(IV)

Z simultaneously being a group of the formula V

$$-COCl \qquad (V)$$

or Y is a group of the formula V and Z is simultaneously a group of the formula IV.

6. The process as claimed in claim 5, wherein the reaction is carried out in the presence of an acid-binding agent in an inert solvent at a temperature of from 20 to 200°C.

7. Use of the compounds according to claims 1 to 4 as optical brighteners.